(19) Europäisches Patentamt / European Patent Office / Office européen des brevets

(11) **EP 3 610 921 A1**

(12) # EUROPEAN PATENT APPLICATION

(43) Date of publication:
**19.02.2020 Bulletin 2020/08**

(51) Int Cl.:
***A61N 5/06*** *(2006.01)*

(21) Application number: **19190434.1**

(22) Date of filing: **07.08.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.08.2018 KR 20180094912
30.10.2018 KR 20180131099**

(71) Applicant: **Metacine, Inc.
Wonju-si, Gangwon-do 26493 (KR)**

(72) Inventor: **PARK, Bae Keun
26494 Gangwon-do (KR)**

(74) Representative: **Ström & Gulliksson AB
P O Box 4188
203 13 Malmö (SE)**

(54) **RHINITIS PHOTO THERAPEUTIC DEVICE WITH A NASAL PROBE USING LOW-LEVEL LASER BASED ON DIFFUSING LIGHT TECHNOLOGY**

(57)     There is disclosed a rhinitis photo therapeutic device with a nasal probe using a low-level laser based on diffusing light technology. In the rhinitis photo therapeutic device with a nasal probe using a low-level laser based on diffusing light technology of the invention, the probe has a configuration in which a light source using a low-level laser is installed, and laser light emitted from the light source is diffused through a lens, and the light is guided through a total reflection prism. In this manner, the rhinitis photo therapeutic device has an effect of relieving inflammation and pain in a nasal cavity by using low-level laser therapy (LLLT).

EP 3 610 921 A1

## Description

BACKGROUND OF THE INVENTION

Field of the Invention

The present invention relates to a rhinitis photo therapeutic instrument, more specifically, a rhinitis photo therapeutic device with a nasal probe using a low-level laser based on diffusing light technology, which is capable of irradiating an inside of a nasal cavity in a wide range and performing laser acupuncture in parallel in order to enhance a therapeutic effect, in a structure in which it is possible to diffuse laser light to every position in the nasal cavity.

Description of the Related Art

The level of living has improved generally due to economic development; however, an incidence rate of a disease such as allergic rhinitis has increased as environmental pollution, particularly, air pollution, becomes more serious.

A research report shows that an increase rate of patients with allergic rhinitis due to such genetic and environmental factors is 15% or higher (research results from 2009 to 2013), and an increase rate of medical expenses by patients with allergic rhinitis is an average of 8.7% or higher per year (research results from 2008 to 2012).

Rhinitis means an inflammatory disease of nasal mucosa, which is accompanied by one or more symptoms of nasal mucus, sneezes, pruritus, and nasal congestion. Acute rhinitis is infectious rhinitis that is called a cold generally, and recurrent rhinitis due to incomplete therapy of the acute rhinitis can develop bacterial-induced chronic rhinitis. Most of chronic rhinitis patients complain about a nasal congestion symptom. When the nasal congestion continues, the patients have a trouble in breathing through their noses and breathe through their mouths, and thus the patients become vulnerable to pharyngolaryngitis and often have a sore throat. In addition, the nasal congestion is likely to result in distraction of attention and a decrease in concentration due to a headache and thus, can result in considerable disruption in work and study.

It is obvious that the patients have a tendency to distrust western/oriental medicine (a temporary medicinal effect or a side effect by long-term medication) and there is an increase in preference for a non-surgical therapy (worry about a burden of a rhinitis surgery cost and the side effect) in a therapy of rhinitis.

Equipment used for a non-surgical rhinitis therapy treats an inflammation by mainly irradiating an affected site in a nasal cavity with LED light having a specific wavelength bandwidth or laser light. Such a light irradiator uses a single light source of red light or blue light; however, the light source has a different effect for each wavelength bandwidth, and thus there is a demand for a light irradiator that is capable of using light having various wavelength bandwidths.

In addition, in a case where rhinitis is treated by one method of using light, a rhinitis therapy effect is small, the light does not transmit into a turbinal among sites in the nasal cavity in which inflammation occurs, and it is difficult to perform a physical therapy. Hence, there is a demand for a rhinitis photo therapeutic device configured to perform two or more complex functions in order to improve therapy to the maximum extent.

Meanwhile, a technology of laser medical equipment is evolved with the advent of new light sources, and examples of a light source that is widely commercialized recently include a fiber laser, a laser diode (LD), a light emitting diode (LED), an organic LED (OLED), a surface-emitting plastic foil, or the like.

As the development of the various light sources widens a range of use of the laser medical equipment from a general hospital to a private hospital and home and increases a possibility of creating a new market, small businesses in Korea is rapidly developing various delicate therapeutic laser development technologies in difficult conditions.

The laser therapy technologies have a high possibility of success only when the development progresses reflect a clinical application that is realized only by using elements constituting multi-function equipment simultaneously, such as improvement of a complex therapy method or a conventional therapy method, finding of a new indication, or the like.

Here, a rhinitis photo therapeutic instrument is limited to a function of irradiating only a local region in the nasal cavity and uses a light irradiation therapeutic method that does not reflect an important anatomical therapy point such as a mucosal tissue region in the nasal cavity, and thus it is necessary to overcome the limit and develop a product having differentiated competitiveness.

In particular, the inside of the nasal cavity is a significantly broader space, compared with a size of a small nostril in which a laser nozzle is positioned. The inside of the nose includes upper, middle, and lower nasal conchas and a wide region of a mucosal layer is formed, the wide nasal mucosa generates mucilaginous rhinorrhea continuously, in which a phenomenon of swelling of nasal mucosa, a change in color and viscosity of rhinorrhea, or the like due to an allergic reaction or a bacterial infection occurs.

The mucosal layer, from which rhinorrhea can be generated, is not only formed on a nasal cavity wall but also in a so-called paranasal cavity as a schematic view of several small spaces positioned on the periphery of eyes, a nose, and a brain. The paranasal cavity also generates rhinorrhea from internal mucosa thereof and discharges foreign matter outside through ciliary movement.

The paranasal cavity is characterized by having a very narrow passage that connects the inside of the nose to the paranasal cavity. When a wall of the passage swells due to bacterial infection or the like, air circulation is blocked, it is not possible to discharge mucus secretion, so rhinorrhea is accumulated and bacteria breed. Then, this ends up as empyema that causes a headache and a bad smell.

A conventional laser product for treating these symptoms irradiates a local region, similarly to a surgical laser that irradiates a target region with intensive laser light by using straightness and monochrome of the laser light so as to remove the corresponding region, and thus a problem arises in that therapy efficiency decreases.

Citation List

Patent Literature

Patent Literature 1: Korean Patent No. 10-1567946 (November 04, 2015)

SUMMARY OF THE INVENTION

In order to solve the problem, an object of the invention is to provide a rhinitis photo therapeutic probe using a low-level laser based on diffusing light technology with which it is possible to relieve inflammation and pain in a nasal cavity by using a low-level laser therapy (LLLT).

In addition, another object of the invention is to provide a rhinitis photo therapeutic probe using a low-level laser based on diffusing light technology which is equipped with an efficient laser light diffusing structure achieved by reflecting a broader internal structure of a nose instead of a small nostril or a local region.

In addition, still another object of the invention is to provide a rhinitis photo therapeutic probe using a low-level laser based on diffusing light technology which is capable of effectively treating rhinitis by using dual wavelengths.

Also, still another object of the invention is to provide a rhinitis photo therapeutic probe using a low-level laser based on diffusing light technology which is capable of irradiating the inside of the nasal cavity in a wide range and performing laser acupuncture in parallel in order to enhance an effect.

In order to solve the problem, according to an embodiment of the invention, there is provided a rhinitis photo therapeutic device with a nasal probe using a low-level laser based on diffusing light technology in which the probe has a configuration in which a light source using a low-level laser is installed, and laser light emitted from the light source is diffused through a lens, and the light is guided through a total reflection prism.

The probe includes a pair of laser diode covers that each has a inner surface provided with multiple semicircular recesses for inducing light diffusion with a transparent structure, a lens that is inserted in each of the laser diode cover and diffuses light emitted from a laser diode, a pair of nose clamps that presses a columella nasi, a probe cover in which the pair of laser diode covers is inserted, and the pair of nose clamps is inserted between the laser diode covers, an inner cover which is joined to a lower portion of the probe cover so as to support the nose clamp and in which the laser diode is inserted, and a lower cover that is joined to the probe cover.

In addition, the nose clamp is formed of an elastic material into a forceps shape so as to press and fix the columella nasi from both sides, and a part of the nose clamp, which presses the columella nasi, partially projects such that an end portion of the part faces a direction opposite to a pressed part.

The lens is configured of a concave lens, and laser light primarily diffused by the concave lens is secondarily diffused by the semicircular recesses formed on the inner surface of the laser diode covers.

The rhinitis photo therapeutic device includes: a laser beam emitting unit to which electric power is applied and which outputs a laser beam having a wavelength of 650 nm and/or a laser beam having a wavelength of 905 nm simultaneously; a key input unit that has an ON/OFF button for turning on and off a laser beam emitting operation of the laser beam emitting unit, a mode selecting button, and a level selecting button; and a control unit that transmits a laser beam emitting operation ON/OFF signal to the laser beam emitting unit in response to a key input by the key input unit and performs control such that the laser beam emitting unit emits the laser beam at a set level for a set operation time.

It is preferable that the lens is configured to have a reflective surface installed at an angle of 45° with a position of the diode as a reference such that incident light to the reflective surface is totally reflected. It is more preferable that the lens has a configuration in which the incident light is totally reflected in one direction toward any one of a position on a furrow of a rounded side of the nose, a center of a side edge of the rounded side of the nose, or a nasal mucosa.

In order to solve the problem, according to an embodiment of the invention, there is provided a rhinitis photo therapeutic device with a nasal probe using a low-level laser based on diffusing light technology in which the probe has a configuration in which a light source using a low-level laser is installed, and laser light emitted from the light source is diffused through a lens, and the light is guided through a total reflection prism.

The probe includes a pair of laser diode covers that each has a surface provided with multiple semicircular projections for inducing light diffusion with a transparent structure, a lens that is inserted in each of the laser diode cover and diffuses light emitted from a laser diode, a pair of nose clamps that presses a columella nasi, a probe cover in which the pair of laser diode covers is inserted, and the pair of nose clamps is inserted between the laser diode covers, an inner cover which is joined to a lower portion of the probe cover so as to support the nose clamp and in which the laser diode is inserted, and a lower cover that is joined to the probe cover.

In addition, the nose clamp is formed of an elastic material into a forceps shape so as to press and fix the columella nasi from both sides and is configured to have a part for pressing the columella nasi, which partially projects such that an end portion of the part faces a direction opposite to a pressed part.

The lens is configured of a concave lens, and laser light primarily diffused by the concave lens is secondarily diffused by the semicircular projections formed on the surface of the laser diode covers.

The rhinitis photo therapeutic device includes: a laser beam emitting unit to which electric power is applied and which outputs a laser beam having a wavelength of 650 nm and a laser beam having a wavelength of 905 nm simultaneously; a key input unit that has an ON/OFF button for turning on and off a laser beam emitting operation of the laser beam emitting unit, a mode selecting button, and a level selecting button; and a control unit that transmits a laser beam emitting operation ON/OFF signal to the laser beam emitting unit in response to a key input by the key input unit and performs control such that the laser beam emitting unit emits the laser beam at a set level for a set operation time.

It is preferable that the lens is configured to have a reflective surface installed at an angle of 45° with a position of the diode as a reference such that incident light to the reflective surface is totally reflected. It is more preferable that the lens has a configuration in which the incident light is totally reflected in one direction toward any one of a position on a furrow of a rounded side of the nose, a center of a side edge of the rounded side of the nose, or a nasal mucosa.

BRIEF DESCRIPTION OF THE DRAWINGS

Fig. 1 is a diagram of a main configuration of a rhinitis photo therapeutic device with a nasal probe using a low-level laser based on diffusing light technology according to an embodiment of the invention;
Fig. 2 is a perspective view of the rhinitis photo therapeutic device of the invention;
Fig. 3 is a perspective view of a main body of the rhinitis photo therapeutic device of the invention;
Fig. 4 is an exploded perspective view of a probe of the invention;
Fig. 5 is a reference diagram for describing a lens assembled in the probe and light diffusion and refraction by the lens;
Fig. 6 is a diagram illustrating a laser diode cover that has a surface provided with multiple semicircular recesses;
Fig. 7 is a graph illustrating a refractive index in a case of wavelengths of 650 nm and 905 nm based on a refractive index of a PC material; and
Figs. 8 and 9 are diagrams illustrating acupuncture points.

DETAILED DESCRIPTION OF THE PREFERRED EMBODIMENTS

A term or word, which is used in this specification or What is claimed is, is not construed in a limited meaning of a common or dictionary meaning and has to be construed in a meaning an a concept in accordance with a technical idea of the invention based on the principle that the inventor can appropriately define a concept of a term in order to describe his or her invention in the best way.

In the entire specification, when a certain part "includes" a certain configurational element, this means that other configurational elements are not excluded but the configurational element can be further included unless specifically described otherwise. In addition, a term of "unit", "section", "module", "device" or the like described in the specification means a basic unit that executes at least one of a function or an operation, and an element assigned with the term can be implemented in a combination of types of hardware and/or software.

In the entire specification, the word "and/or" has to be construed to include every providable combination of one or more related items. For example, "a first item, a second item, and/or a third item" means every providable combination of two or more items of the first, second, and third items, as well as the first, second, or third item.

In the entire specification, an identification sign (for example, a, b, c, or the like) at each of stages is used for convenience of description, and the identification sign does not limit an order of the stages. The stages may occur in a different order from a described order unless a specific order is clearly described in context. That is, the stages may occur in the same order as the described order, may occur substantially at the same time, or may occur in a reverse order.

Hereinafter, an embodiment of the invention will be described with reference to the drawings.

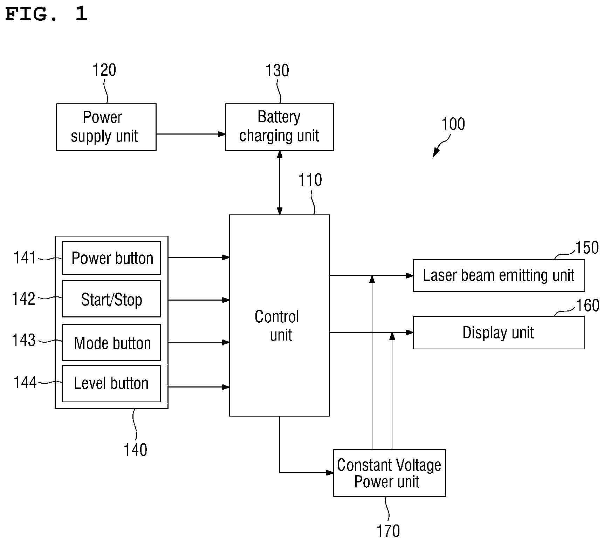

Fig. 1 is a diagram of a main configuration of a rhinitis photo therapeutic device with a nasal probe using a low-level laser based on diffusing light technology according to an embodiment of the invention. The invention does not use intensive laser light that destroys tissue as by a surgical laser instrument but uses low-level laser light by which a low-level laser therapy (LLLT) with a biomedical stimulation effect is performed by transmitting light energy through skin tissue such that the light energy directly influences the tissue. The invention is characterized in that dual-wavelength laser light having wavelengths of 650 nm and/or 905 nm is used.

That is, the invention is a rhinitis photo therapeutic device that irradiates a local region in a nasal cavity with two beams of low-level laser light having different or same wavelengths from each other and relieves pain and a rhinitis symptom.

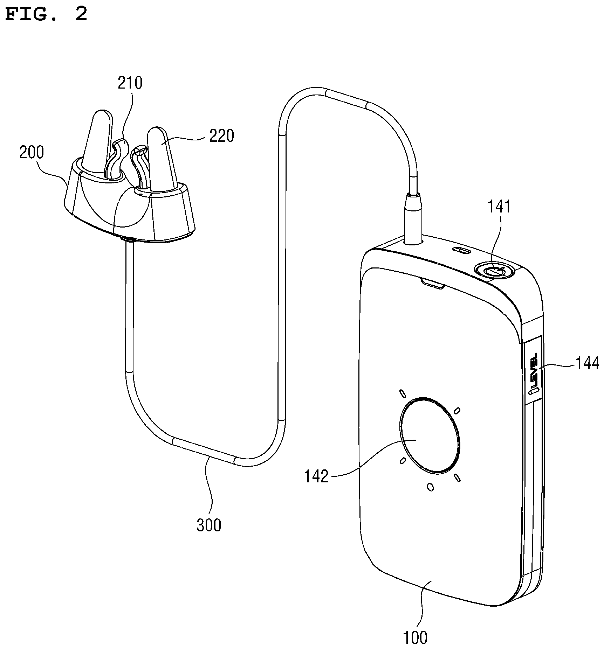

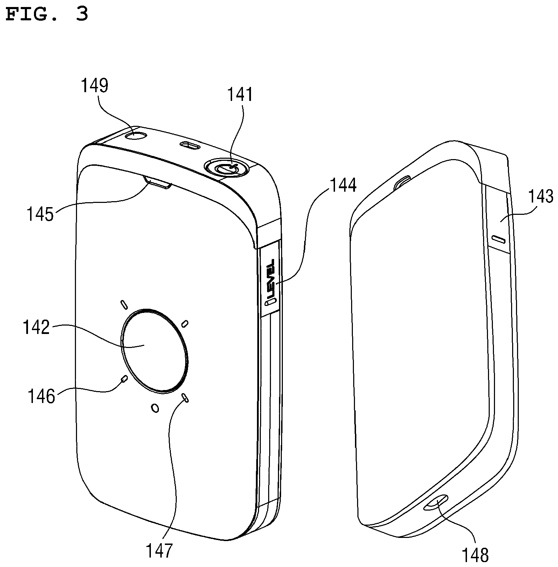

With reference to a perspective view of the rhinitis photo therapeutic device of the invention in Fig. 2 and a perspective view of a main body of the rhinitis photo therapeutic device of the invention in Fig. 3, the rhinitis photo therapeutic device of the invention is configured to include a main body 100 that emits laser light, a probe 200 using a low-level laser based on diffusing light technology, and a cable 300 that connects the main body 100 and the probe 200.

First, the main body 100 includes a laser beam emitting unit 150 to which electric power is applied from a power supply unit 120 and which outputs a laser beam having a wavelength of 650 nm and/or a laser beam having a wavelength of 905 nm simultaneously, a key input unit 140 that has a power button 141 for controlling ON/OFF of power, a start/stop button 142 for turning on and off a laser beam emitting operation of the laser beam emitting unit 150, and a level button 144 for selecting a level, and a control unit 110 that transmits a laser beam emitting operation ON/OFF signal to the laser beam emitting unit 150 in response to a key input by the key input unit 140 and performs control such that the laser beam emitting unit 150 emits the laser beam at a set level for a set operation time.

In addition, the main body 100 may further include a probe connecting terminal 149 to which a connection jack of the probe 200 is connected, a power/charge LED 145 that indicates a power state of a product, an emission intensity selecting LED 147, a laser irradiation mode LED 146, a charge terminal 148, or the like

As one characteristic, the probe 200 has a configuration in that a low-level laser light source is installed, laser light emitted from the light source is diffused through a lens, and the light is guided through a total reflection prism such that inflammation and pain in the nasal cavity are relieved, and the probe is equipped with an efficient laser light diffusing structure so as to be capable of effectively treating rhinitis by using dual wavelengths.

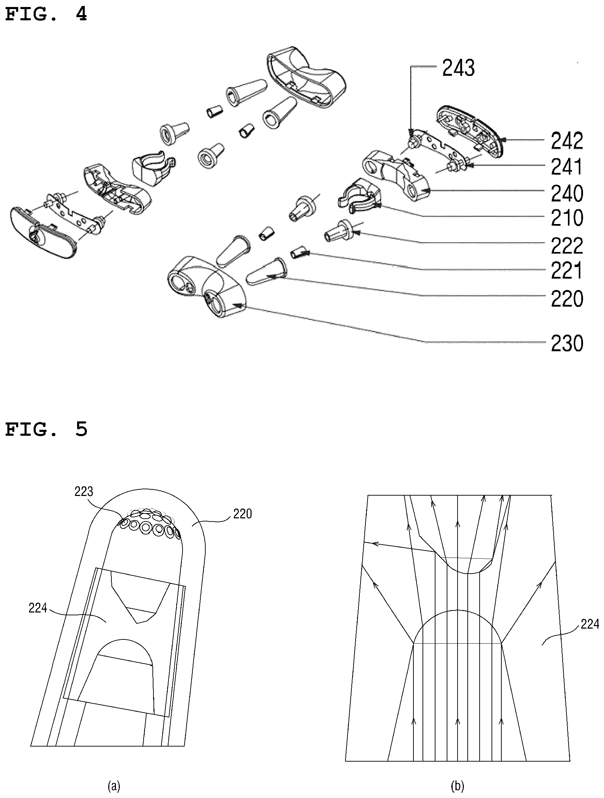

With reference to an exploded perspective view of the probe of the invention in Fig. 4, the probe 200 is configured to include a pair of laser diode covers 220 that each has a surface provided with multiple semicircular recesses for inducing light diffusion with a transparent structure, a laser reflecting lens 221 that is inserted in each of the laser diode covers 220 and diffuses light emitted from a laser diode 243, a pair of nose clamps 210 that presses a columella nasi, a probe cover 230 in which the pair of laser diode covers 220 is inserted and the pair of nose clamps 210 is inserted between the laser diode covers 220, a PCB fixed cover 240 which is joined to a lower portion of the probe cover 230 so as to support the nose clamps 210 and in which the laser diode 243 is inserted, and a lower cover 242 that is joined to the probe cover 230.

The nose clamp 210 is formed of an elastic material into a forceps shape so as to press and fix the columella nasi from both sides, and a part of the nose clamp, which presses the columella nasi, partially projects such that an end portion of the part faces a direction opposite to a pressed part.

In addition, the lens 221 is a concave lens and is configured to cause laser light primarily diffused by the concave lens to be secondarily diffused by the semicircular recesses formed on the inner surface of the laser diode covers 220.

In addition, It is preferable that the lens is configured to have a reflective surface installed at an angle of 45° with a position of the diode as a reference such that incident light to the reflective surface is totally reflected, and the lens has a configuration in which the incident light is totally reflected in one direction toward any one of a position on a furrow of a rounded side of the nose, a center between side edges of the rounded sides of the nose, or a nasal mucosa.

The nose clamp 210 is formed of a silicon material as a harmless component to a human body, is configured to have a forceps shape so as to press and fix the columella nasi of a human body by a method in which the product is fixed to the nose, and is formed of a soft and elastic material so as to reduce irritability. In particular, the nose clamp is configured to have an upper end that is finished to face outward such that a wearer can have a minimum pressed feeling.

That is, the nose clamp is configured to have the part for pressing the columella nasi, which partially projects such that the end portion of the part faces the direction opposite to the pressed part.

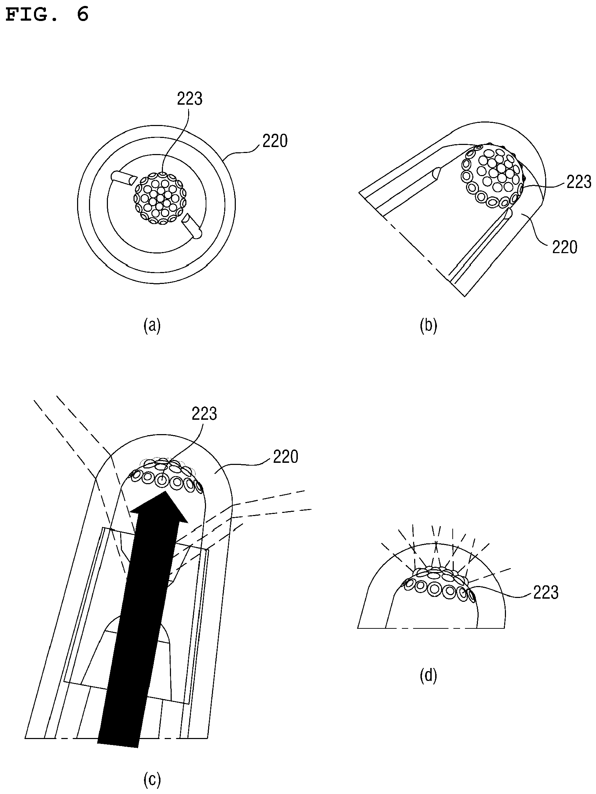

In addition, with reference to the drawing illustrating the laser diode cover that has the inner surface provided with the multiple semicircular recesses in Fig. 6, the semicircular recesses 223 formed in the laser diode cover 220 are configured to be recessed as 32 small hemispheres so as to irradiate every corner in the nose with the laser light.

With reference to the drawing, even when the laser light is diffused through the concave lens 224, the light is still irradiated more intensively in the center direction (an arrow of (c)). Therefore, the 32 small semicircular recesses 223 are to play a role of diffusing intensive beams at the center to every corner in the nose.

That is, the lower drawing shows that the 32 small semicircular recesses are formed on an inner surface of an upper end of the diode cover that is an external structure of the lens such that light refraction is induced.

A schematic diagram of a light diffusing structure in which light is concentrated at the center as illustrated in the lower left drawing shows that each of the hemispheres formed as illustrated in the right drawing diffuses light such that the irradiation is performed in the inside of the nasal cavity in a wide range.

As described above, the invention has another characteristic of using the concave lens and the 32 semicircular recesses 223 in order to diffuse light by ergonomic design obtained by reflecting the nasal cavity structure.

A conventional laser product for treating these symptoms irradiates a local region, similarly to a surgical laser that irradiates a target region with intensive laser light by using straightness and monochrome of the laser light so as to remove the corresponding region, and thus a problem arises in that therapy efficiency decreases

Fig. 5 illustrates the lens assembled in the nasal probe and diffusion and refraction of light by the lens. The schematic diagram in the left illustrates a nasal probe projections in which the lens is assembled, and the right diagram illustrates light diffusion and refraction by the lens.

The concave lens 224 primarily diffuses the laser light generated by the laser diode, and 32 small semicircular recesses 223 on the diode cover is to secondarily diffuse the light significantly concentrated relatively at the center portion.

In addition, when the refraction of the laser light is estimated, reflection is performed in a condition of 0 > 40°.

The right drawing is a schematic diagram illustrating a laser light diffusing path of a product of the invention, which is obtained through consultation with optical physics professors. It is shown that much refraction and reflection (diffused reflection, total reflection, or the like) is performed.

Advantages of the laser light is that it has a constant wavelength when the light diffusion occurs due to monochrome and straightness, and thus it is possible to set the direction of the light by using a structure and to quantify the light.

The invention is designed to primarily induce a refraction by the lens, and then to induce secondary refraction and total reflection at a surrounding structure. That is, the light is dispersed by the lens to the right and left sides at an angle of 45° or larger and is guided such that a part of the light is totally reflected by using a lens structure to a parget position, and the lens has the diode cover so as to cause diffused reflection of the laser light.

The total reflection is a phenomenon in which every light is reflected in a case where an incident angle becomes bigger than a critical angle when light travels straightly from a substance having a larger refractive index toward a substance having a smaller refractive index. The total reflection is induced by installing a reflective surface at an angle of 45° with the position of the diode as a reference in order to irradiate a target acupuncture point with light.

A reflection rate is 8 to 2, and about 20% of light is reflected.

When the reflection rate is described with respect to a diameter of the laser diode, the reflection rate accounts for 20% of the diameter, and, in order to cause an amount of light to travel to an area corresponding to 20% of an area of the diode, Expression 1 is applied. It is needless to say that the value is a value obtained by simple calculation, and thus the value can be different from an actually measured value.

```
Formula 1

[(Total      reflection-structured      surface)      ×
cos45]/diameter of diode = 0.2
```

Accordingly, when the laser is emitted by 5 mW in the invention, light corresponding to 20% of light traveling to a reflective surface of the acupuncture point will be 1 mW or lower.

In order to stimulate the acupuncture point, a depth of an acupoint needs to be estimated, and an average depth of 361 acupoints is approximately 0.48 inches, corresponding to about 1.1 to 1.5 cm.

Accordingly, in the rhinitis photo therapeutic device of the invention, GaAs laser light having a wavelength of 905 mm has permeability of 30 to 50 mm. This means a sufficient possibility of having a biomedical effect.

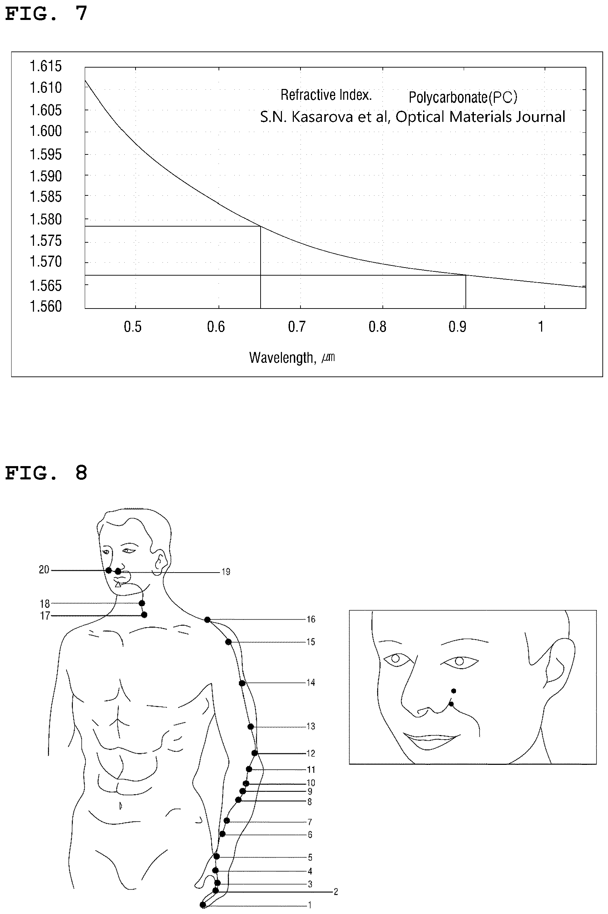

With reference to a diagram illustrating acupuncture points in Fig. 8, a representative acupuncture point used for a rhinitis therapy is "Young-Hyang" (LI-20) which is positioned at each of the centers on edges of the right and left round sides of the nose. In the Korean Standards (KS), the "Young-Hyang" (LI-20) is described to have the same height as the median points of corners of edges of the rounded sides of the nose, top of the furrows of the rounded sides of the nose, and the face and is a acupuncture point belonging to a channel called "Soo-Yang-Myeong-Dae-Jang-Kyeong" of twelve channels, and acupuncture is performed on the point for a patient suffering from rhinitis.

In addition, upper Young-Hyang (Ex-HN-8) and inner Young-Hyang (Ex-HN-9) are acupuncture points which do not belong to fourteen pulse spots and are acupuncture points which have been found with experience and known to have an effect. Therefore, it is not possible to interpret an action and an indication by different positions and different meridian theory; however, there are many acupuncture points that are known to have a high therapy effect.

The upper Young-Hyang is referred to as a splenic channel acupuncture point, and the inner Young-Hyang is positioned to be opposite to the upper Young-Hyang, that is, on the side of the mucosa of the nose.

The acupuncture points, which can be stimulated when the probe 200 is inserted, are the upper Young-Hyang (splenic channel acupuncture point) that is positioned at a short distance and the inner Young-Hyang. In general, the acupuncture is implemented to the Young-Hyang and the upper Young-Hyang and is not implemented in a case of the inner Young-Hyang. This is because the inner Young-Hyang is positioned in an inner mucosa of the nose and the acupuncture is implemented by penetrating skin, thus it is possible to cause a severe pain and slight bleeding.

However, the laser acupuncture can be implemented without such a problem and thus, is a useful therapy.

By using advantages of the laser acupuncture by which it is possible to diffuse the light to the entire region in the nasal cavity through the irradiation of the nasal cavity by the probe with the laser light once and to cause biomedical stimulation with low-level laser emission, the therapeutic efficacy is to be maximized by guiding a part of total amount of light to rhinitis acupuncture points. In this respect, the invention uses a total reflection prism structure in order to guide the light to the rhinitis acupuncture points.

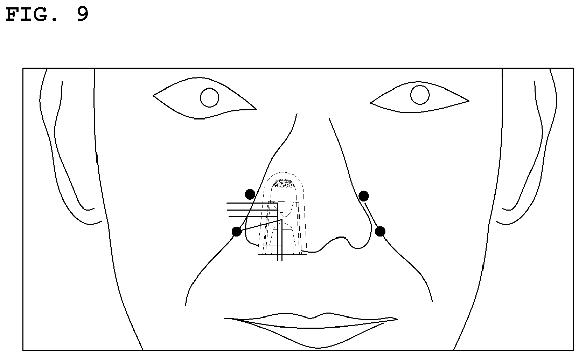

Fig. 9 illustrates an example that shows that it is possible to irradiate right and left outer acupuncture points of the nose with an amount of 20% of the total amount of light totally reflected in a lens structure. When the probe is inserted into the nose, the light can stimulate every acupuncture point that is symmetrically positioned on both sides of the nose.

With reference to a graph illustrating a correlation between refractive indexes of the wavelengths of 650 nm and/or 905 nm based on the refractive index of a PC material, the refractive index is shown to change depending on each wavelength; however, a difference therebetween is not large when an actual application is performed.

The rhinitis photo therapeutic device with a nasal probe using a low-level laser based on diffusing light technology of the invention with the configuration described above operates the control unit 110 by electric power rectified through the power supply unit 120 and is operated to control the laser beam emitting unit 150 by manipulation of the key input unit 140 by a user.

That is, the laser light is emitted depending on a set stage and is emitted to a nasal cavity.

In the invention, the power supply unit 120 uses AC 100 to 240 V, 50 to 60 Hz, and 12 VA, and a battery charging unit 130 is configured of DC 3.7 V, 1,130 mAh, and Li-ion.

Note that a laser grade is 3R in accordance with IEC 60825-1 (2007), and InGaAs/GaAs, which is a semiconductor laser classification by a medium used, is used.

Software installed in the control unit 110 is configured of a structure of a setting module and an operation module. When an ON/OFF button is pressed on the setting module, LED light is turned on in order for the user to check a device operation status, and the operation module includes functions of emitting the laser light and a frequency and showing an operation start and an operation end.

In addition, the device has effects of maximizing the therapy effect (for example, being capable of causing therapy light to reach a deep position in the nasal cavity), achieving convenience of use (for example, enabling a patent to breathe during therapy or preventing nasal mucus from running down during therapy), reducing a side effect (for example, preventing the eye from being irradiated with light), or the like.

In the rhinitis photo therapeutic device, first, the connection jack of the probe 200 is connected to the probe connecting terminal on an upper surface of the main body, and electric power is applied by using the power button 141.

It is preferable that the LED is turned on by pressing the power button for three seconds.

In this case, the LED can indicate statuses, with blue indicating power ON, red flashing indicating low battery, red light indicating charging, and green indicating full charge.

A use mode is set by using a mode button 143 on a left side of the main body. Whenever the button is pressed, the mode is changed rotationally, and each LED is turned on.

When mode selection is completed, a level is set by using the level button.

When the level is set, the probe 200 is inserted to the nasal cavity and is fixed not to be moved in a state in which portions of the probe, on which L and S are inscribed, are viewed by the naked eye. Then, an operation button is pressed such that irradiation with laser light is performed.

After a five-minute operation is ended, the probe is pulled out from the nose. Then, the probe is inserted with switching the left and right sides, and another five-minute operation is performed.

When the operation is completed afterwards, the probe is removed from the nose.

As described above, the commercially available rhinitis therapy instrument is limited to a function of irradiating only a local region in the nasal cavity and uses a light irradiation therapeutic method that does not reflect an important anatomical therapy point such as a mucosal tissue region in the nasal cavity, whereas the rhinitis photo therapeutic device of the invention can be classified as a product that overcomes the limit and has differentiated competitiveness.

Hence, in the rhinitis photo therapeutic device with a nasal probe using a low-level laser based on diffusing light technology according to an embodiment of the invention, it is possible to relieve inflammation and pain in a nasal cavity by using a low-level laser therapy (LLLT).

In addition, the rhinitis photo therapeutic device with a nasal probe using a low-level laser based on diffusing light technology of the invention has an effect of effectively treating rhinitis by using dual wavelengths.

In addition, the rhinitis photo therapeutic device with a nasal probe using a low-level laser based on diffusing light technology of the invention is capable of effectively diffusing the laser light such that a cell is directly irradiated with the laser light, thereby having a relieving effect and an effect of inducing recovery of an inflamed cell.

Also, the rhinitis photo therapeutic device with a nasal probe using a low-level laser based on diffusing light technology of the invention has an effect of inducing a laser acupuncture effect and replacing acupuncture with the laser acupuncture.

As described above, the specific examples described in the invention are described in detail; however, it is obvious for those skilled in the art that it is possible to perform various alterations and modifications within a scope of the technical ideas of the invention, and it is needless to say that the alterations and modifications belong to What is claimed is.

**Claims**

1. A rhinitis photo therapeutic device with a nasal probe using a low-level laser based on diffusing light technology, wherein the probe has a configuration in which a light source using a low-level laser is installed, and laser light emitted from the light source is diffused through a lens, and the light is guided through a total reflection prism.

2. The rhinitis photo therapeutic device with a nasal probe using a low-level laser based on diffusing light technology according to claim 1,
   wherein the probe includes
   a pair of laser diode covers that each has a inner surface provided with multiple semicircular recesses for inducing light diffusion with a transparent structure,
   a lens that is inserted in each of the laser diode covers and diffuses light emitted from a laser diode,
   a pair of nose clamps that presses a columella nasi, a probe cover in which the pair of laser diode covers is inserted, and the pair of nose clamps is inserted between the laser diode covers,
   an inner cover which is joined to a lower portion of the probe cover so as to support the nose clamps and in which the laser diode is inserted, and
   a lower cover that is joined to the probe cover.

3. The rhinitis photo therapeutic device with a nasal probe using a low-level laser based on diffusing light technology according to claim 2,
   wherein each of the nose clamps is formed of an elastic material into a forceps shape so as to press and fix the columella nasi from both sides, and a part of the nose clamp, which presses the columella nasi, partially projects such that an end portion of the part faces a direction opposite to a pressed part.

4. The rhinitis photo therapeutic device with a nasal probe using a low-level laser based on diffusing light technology according to claim 2,
   wherein the lens is a concave lens, and laser light primarily diffused by the concave lens is secondarily diffused by semicircular recesses formed on an inner surface of the laser diode cover.

5. The rhinitis photo therapeutic device with a nasal probe using a low-level laser based on diffusing light technology according to claim 1, the rhinitis photo therapeutic device comprising:

   a laser beam emitting unit to which electric power is applied and which emits a laser beam having a wavelength of 650 nm and/or a laser beam having a wavelength of 905 nm simultaneously;
   a key input unit that has an ON/OFF button for turning on and off a laser beam emitting operation of the laser beam emitting unit, a mode selecting button, and a level selecting button; and
   a control unit that transmits a laser beam emitting operation ON/OFF signal to the laser beam emitting unit in response to a key input by the key input unit and performs control such that the laser beam emitting unit emits the laser beam at a set level for a set operation time.

6. The rhinitis photo therapeutic device with a nasal probe using a low-level laser based on diffusing light technology according to claim 1,
   wherein the lens is configured to have a reflective surface installed at an angle of 45° with a position of the diode as a reference such that incident light to the reflective surface is totally reflected.

7. The rhinitis photo therapeutic device with a nasal probe using a low-level laser based on diffusing light technology according to claim 6,
   wherein the lens has a configuration in which the incident light is totally reflected in one direction toward any one of a position on a furrow of a rounded side of the nose, a center of a side edge of the rounded side of the nose, or a nasal mucosa.

FIG. 1

FIG. 2

## FIG. 3

FIG. 4

243

242
241
240
210
222
221
220
230

FIG. 5

223

220

224

(a)

224

(b)

FIG. 6

(a)

(b)

(c)

(d)

# FIG. 7

# FIG. 8

FIG. 9

Europäisches
Patentamt

European
Patent Office

Office européen
des brevets

# EUROPEAN SEARCH REPORT

Application Number

EP 19 19 0434

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KR 100 836 025 B1 (JEONG SEUNG WON [KR]) 9 June 2008 (2008-06-09) <br> * figures * <br> * paragraph [0026] * <br> * paragraph [0022] * <br> * paragraph [0050]; claim 6 * <br> * paragraph [0058] * <br> * paragraph [0039] * <br> ----- | 1-7 | INV. A61N5/06 |
| Y | US 2016/129278 A1 (MAYER ESTHER [IL]) 12 May 2016 (2016-05-12) <br> * figures 2,3 * <br> * paragraph [0017] * <br> * paragraph [0019] * <br> * paragraph [0027] * <br> * paragraph [0084] * <br> * paragraph [0085] * <br> * paragraph [0088] * <br> * paragraph [0089] * <br> * paragraph [0097] * <br> * paragraph [0032] * <br> * paragraph [0068] * <br> * paragraph [0125] * <br> ----- <br> -/-- | 1-7 | |

| | | | TECHNICAL FIELDS SEARCHED (IPC) |
|---|---|---|---|
| | | | A61N |

The present search report has been drawn up for all claims

| Place of search | Date of completion of the search | Examiner |
|---|---|---|
| Munich | 1 October 2019 | Rodríguez Cosío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches
Patentamt

European
Patent Office

Office européen
des brevets

**EUROPEAN SEARCH REPORT**

Application Number

EP 19 19 0434

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2007/219600 A1 (GERTNER MICHAEL [US] ET AL) 20 September 2007 (2007-09-20)<br>* paragraph [0026] *<br>* paragraph [0048] *<br>* paragraph [0049] *<br>* paragraph [0059] *<br>* paragraph [0069] *<br>* paragraph [0076] *<br>* paragraph [0085] *<br>* paragraph [0103] *<br>* paragraph [0108] *<br>* paragraph [0109] *<br>* paragraph [0116] *<br>* paragraph [0113] *<br>* paragraph [0119] *<br>* paragraph [0120] *<br>* paragraph [0149]; claim 3 *<br>* paragraph [0007] *<br>* paragraph [0003] *<br>* paragraph [0084] *<br>* paragraph [0091] *<br>----- | 1-7 | |
| Y | US 2004/030368 A1 (KEMENY LAJOS [HU] ET AL) 12 February 2004 (2004-02-12)<br>* figures 6-9 *<br>* paragraph [0052] *<br>* paragraph [0057] *<br>* paragraph [0056] *<br>* paragraph [0060] *<br>* paragraph [0073] *<br>* paragraph [0074] *<br>* paragraph [0075] *<br>* paragraph [0076] *<br>-----<br>-/-- | 1-7 | TECHNICAL FIELDS SEARCHED (IPC) |

The present search report has been drawn up for all claims

| Place of search | Date of completion of the search | Examiner |
|---|---|---|
| Munich | 1 October 2019 | Rodríguez Cosío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 19 19 0434

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2007/135874 A1 (BALA JOHN L [US]) 14 June 2007 (2007-06-14) * paragraph [0009] * * paragraph [0016] * * paragraph [0035] * * paragraph [0037] * * paragraph [0052] * ----- | 1-7 | |
| Y | DE 101 20 629 A1 (LENKE MICHAEL [DE]) 31 October 2002 (2002-10-31) * figure 1 * * paragraph [0001] * * paragraph [0008] * * paragraph [0010] * * paragraph [0030] * * paragraph [0031] * * paragraph [0032] * * figures 1,4,9,12,14 * * paragraph [0044] * * paragraph [0046] * * paragraph [0020] * ----- | 1-7 | |
| Y | FR 779 969 A (LAGROUA HENRI JOSEPH; KORDA PIERRE JULES) 17 April 1935 (1935-04-17) * prisme 19; page 3, right-hand column, line 5 - line 6; figure 7 * ----- | 1-7 | TECHNICAL FIELDS SEARCHED (IPC) |
| Y | KR 101 610 896 B1 (KIM YONG HO [KR]) 20 April 2016 (2016-04-20) * figure 7 * * paragraph [0036]; figures 13,14,15 * * paragraph [0047]; figures 19,20 * * paragraph [0048] * * paragraph [0049] * * paragraph [0057] * * paragraph [0013] * * paragraph [0050] * ----- -/-- | 2-7 | |

The present search report has been drawn up for all claims

| Place of search | Date of completion of the search | Examiner |
|---|---|---|
| Munich | 1 October 2019 | Rodríguez Cosío, J |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

# EUROPEAN SEARCH REPORT

Application Number

EP 19 19 0434

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2009/093865 A1 (KRESPI YOSEF [US] ET AL) 9 April 2009 (2009-04-09)<br>* paragraph [0020] *<br>* paragraph [0045] *<br>* paragraph [0044] *<br>* paragraph [0047] *<br>* paragraph [0049] *<br>* paragraph [0077]; claims 10-12 *<br>* paragraph [0081] *<br>* paragraph [0086] *<br>* paragraph [0107] *<br>----- | 6 | |
| Y | WO 2011/142632 A2 (OPTOWELL CO LTD [KR]; YANG GYE MO [KR]; LEE DUK DONG [KR]) 17 November 2011 (2011-11-17)<br>* paragraph [0133] - paragraph [0140] *<br>----- | 6 | |

TECHNICAL FIELDS
SEARCHED        (IPC)

The present search report has been drawn up for all claims

| Place of search | Date of completion of the search | Examiner |
|---|---|---|
| Munich | 1 October 2019 | Rodríguez Cosío, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 19 0434

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2019

| Patent document cited in search report | | Publication date | Patent family member(s) | | Publication date |
|---|---|---|---|---|---|
| KR 100836025 | B1 | 09-06-2008 | NONE | | |
| US 2016129278 | A1 | 12-05-2016 | CA | 2602835 A1 | 05-10-2006 |
| | | | EP | 1871476 A2 | 02-01-2008 |
| | | | ES | 2668959 T3 | 23-05-2018 |
| | | | US | 2009319008 A1 | 24-12-2009 |
| | | | US | 2013030507 A1 | 31-01-2013 |
| | | | US | 2016129278 A1 | 12-05-2016 |
| | | | WO | 2006103678 A2 | 05-10-2006 |
| US 2007219600 | A1 | 20-09-2007 | US | 2007219600 A1 | 20-09-2007 |
| | | | WO | 2007109496 A2 | 27-09-2007 |
| US 2004030368 | A1 | 12-02-2004 | AT | 430598 T | 15-05-2009 |
| | | | EP | 1420857 A1 | 26-05-2004 |
| | | | ES | 2325058 T3 | 25-08-2009 |
| | | | HU | 0103279 A2 | 28-04-2003 |
| | | | JP | 4245476 B2 | 25-03-2009 |
| | | | JP | 2004537382 A | 16-12-2004 |
| | | | PL | 204588 B1 | 29-01-2010 |
| | | | RU | 2290224 C2 | 27-12-2006 |
| | | | SK | 872004 A3 | 03-08-2004 |
| | | | UA | 84257 C2 | 10-10-2008 |
| | | | US | 2004030368 A1 | 12-02-2004 |
| | | | US | 2004204747 A1 | 14-10-2004 |
| | | | US | 2006111760 A1 | 25-05-2006 |
| | | | US | 2006136019 A1 | 22-06-2006 |
| | | | US | 2006155349 A1 | 13-07-2006 |
| | | | US | 2006235492 A1 | 19-10-2006 |
| | | | WO | 03013653 A1 | 20-02-2003 |
| US 2007135874 | A1 | 14-06-2007 | NONE | | |
| DE 10120629 | A1 | 31-10-2002 | DE | 10120629 A1 | 31-10-2002 |
| | | | EP | 1423164 A1 | 02-06-2004 |
| | | | WO | 02087698 A1 | 07-11-2002 |
| FR 779969 | A | 17-04-1935 | NONE | | |
| KR 101610896 | B1 | 20-04-2016 | KR | 101610896 B1 | 20-04-2016 |
| | | | WO | 2017104976 A1 | 22-06-2017 |
| US 2009093865 | A1 | 09-04-2009 | US | 2009093865 A1 | 09-04-2009 |
| | | | WO | 2008067361 A2 | 05-06-2008 |
| WO 2011142632 | A2 | 17-11-2011 | NONE | | |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 19 19 0434

This annex lists the patent family members relating to the patent documents cited in  the above-mentioned European search report.
The members are as contained in the European Patent Office EDP file on
The European Patent Office is in no way liable for these particulars which are merely given for the purpose of information.

01-10-2019

| Patent document cited in search report | Publication date | Patent family member(s) | Publication date |
|---|---|---|---|

EPO FORM P0459

For more details about this annex : see Official Journal of the European Patent Office, No. 12/82

page 2 of 2

**EP 3 610 921 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101567946